(19) Europäisches Patentamt
European Patent Office
Office européen des brevets

(11) Publication number: **0 371 495 A2**

(12) **EUROPEAN PATENT APPLICATION**

(21) Application number: **89122070.9**

(22) Date of filing: **29.11.89**

(51) Int. Cl.[5]: **C07H 15/203, C07H 15/26, A61K 47/00**

Claims for the following Contracting State: ES.

(30) Priority: **29.11.88 JP 301955/88**

(43) Date of publication of application: **06.06.90 Bulletin 90/23**

(84) Designated Contracting States: **AT BE CH DE ES FR GB GR IT LI LU NL SE**

(71) Applicant: **MECT CORPORATION**
**1-1, Nishi-Shinjuku 2-chome**
**Shinjuku-ku Tokyo(JP)**

(72) Inventor: **Yoshimura, Shoji**
**5-3-7-206, Azumacho**
**Iruma-shi Saitama(JP)**
Inventor: **Tanaka, Makoto**
**760-6, Shimomakuri**
**Koshigaya-shi Saitama(JP)**

(74) Representative: **Leyh, Hans, Dr.-Ing.**
**Patentanwälte Berendt, Leyh & Hering Innere Wiener Strasse 20**
**D-8000 München 80(DE)**

(54) **Sialic acid derivative with active ester groups.**

(57) Sialic acid derivative with active ester groups expressed with the formula [I]

[ I ]

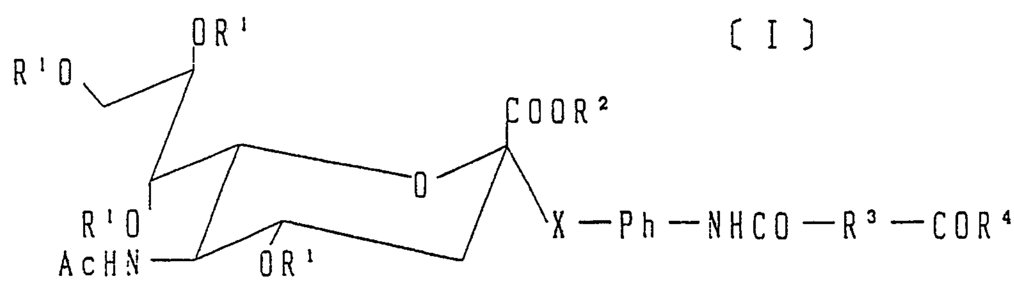

Where $R^1$ denotes hydrogen or acetyl group, $R^2$ hydrogen or lower alkyl group, $R^3$ $C_2H_4$, $C_3H_6$ or $C_2H_2$, $R^4$ hydroxyl group, residue left after removing hydrogen from the alcohol portion of active ester or alkyloxycarbonyloxy group, Ac acetyl group, Ph phenyl group, X oxygen or sulfur. This sialic acid derivative has high reactivity because it has active ester groups in molecules and can be used as raw material or intermediate for synthesis of various sialic acid derivatives.

EP 0 371 495 A2

# Sialic acid derivative with active ester groups

## BACKGROUND OF THE INVENTION

### 1. Field of the Invention

This invention relates to new sialic acid derivatives and more specifically to sialic acid derivatives having active ester groups in the molecules, biochemical half-life extenders of biologically active substances, sialic acid derivatives bonding this sialic acid derivatives with amino compounds, and intermediate compounds used for synthesis of the sialic acid derivatives.

### 2. Related Art Statement

Neuraminic acid derivatives including N-acetylneuraminic acid, that is, sialic acid derivatives, are known to exist widely in the animal world or on the cell surface of several bacteria as sialo complex (glycoprotein, glycolipid, oligosaccharides, and polysaccharides).

The above-mentioned sialic acid derivatives are compounds which are valued highly recently in medical and pharmaceutical fields, such as nervous functions, cancer, inflammation, immunity, virus infection, differentiation, and hormone receptor, and are attraction keen attention as peculiar active molecules locally existing on the cell surface.

Various theories have been put forward on the part played by sialic acid derivatives in the aforementioned sialo complex, but there are so many things that have not yet been clarified, and are still a matter of conjecture.

The inventor has studied the sialic acid derivatives over many years and succeeded in synthesizing sialic acid derivatives which exhibit conspicuous biological activity (Japanese Patent Application No.62-295641).

Recently, the inventor discovered a new sialic acid derivative that exhibits conspicuous biological activity and has realized this invention.

## SUMMARY OF THE INVENTION

Therefore, an object of the present invention is to provide a new sialic acid derivative which exhibits high reactivity with various amino compounds.

Another object of the present invention is to provide biological half-life extenders of various biologically active substances using the said sialic acid derivative.

Still another object of the present invention is to provide a new sialic acid derivative which bonds the said sialic acid derivative to various amino compounds including amino acids and amines through amide bonding.

A further object of the present invention is to provide a new sialic acid derivative which is useful as the intermediate for synthesis of the said sialic acid derivative.

The above and other objects, features, and advantages of the present invention will become more apparent from the following detailed description and embodiments.

The sialic acid derivative of the present invention shall have the active ester groups expressed by the formula [I].

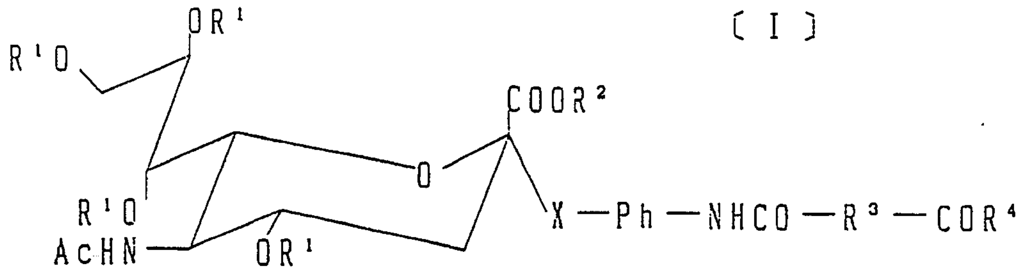

[ I ]

where $R^1$ denotes hydrogen or acetyl group, $R^2$ hydrogen or lower alkyl group, $R^3$ $C_2H_4$, $C_3H_6$ or $C_2H_2$, $R^4$ hydroxyl group, residue left after removing hydrogen from the alcohol portion of active ester or alkyloxycarbonyloxy group, Ac acetyl group, Ph phenyl group, and X oxygen or sulfur.

In the said sialic acid derivative, the residue ($R^4$) left after removing hydrogen from the alcohol portion of the active ester includes N-hydroxysuccinimide, N-hydroxy-5-norbornene-2, 3-dicarboximide, N-hydroxyphthalimide, N-hydroxybenzotriazole, p-nitrophenol, 2, 4-dinitrophenol, 2, 4, 5-trichlorophenol, or pentachlorophenol.

The alkyloxycarbonyloxy group ($R^4$) is introduced by allowing carboxylic acid to react with alkyl halogenoformate in the presence of bases, and the alkyl group includes methyl group, ethyl group, n-butyl group, isobutyl group, phenyl group, and benzyl group.

The sialic acid derivative containing the said ester group is prepared by the following method using N-acetyl neuraminic acid expressed with the following formula as starting material.

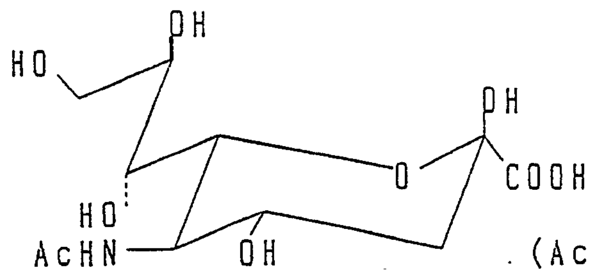

. ($Ac=CH_3CO-$)

where Ac denotes acetyl group. The same applies to the following.

At first, the methyl ester substance is given at a high yield by letting N-acetylneuraminic acid react with methanol in the presence of ion-exchange resin Dowex 50w ($H^+$). Then, the methyl-ester compound is allowed to react with excess acetyl chloride ($CH_3COCl$), then with ethanol white cooling, to give the chlor substance at a high yield. Then, this chlor substance is allowed to react with anhydrous sodium salt of p-nitrophenol in the anhydrous dimethylformamide to give p-nitrophenylglycoside at a high yield. The p-nitrophenylglycoside is a known compound described in the "Carbohydrate Research, 162 (1987) 294 - 297" and the detail of the synthesis method will be discussed in the Embodiment 1 herein.

The said chlor substance is allowed to react with anhydrous sodium salt of p-nitrothiophenol in the anhydrous dimethylformamide to give p-nitrophenylthioglycoside (Embodiment 2). Then, to the aforementioned p-nitrophenylglycoside, hydrogen is added in the presence of 5 % Pd/C in the methanol to give p-aminophenylglycoside (Embodiment 3). Adding hydrogen to the said p-nitrophenylthioglycoside in the presence of 5 % Pd/C in methanol expedites the reductive alkylation reaction and N, N′-dimethylaminophenylthioglicoside is obtained (Embodiment 4). On the other hand, adding hydrogen to the said p-nitrophenylthioglycoside in the acetic acid in the presence of 5 % Pd/C gives p- aminophenylthioglycoside (Embodiment 5). Next, the said p-aminophenylglycoside is allowed to react with slightly excess succinic anhydride in anhydrous tetrahydrofran to give an amido-carboxylic acid compound (Embodiment 6). The said p-aminophenylthioglycoside is allowed to react with slightly excess succinic anhydride in anhydrous tetrahydrofran to give an amido-carboxylic acid compound (Embodiment 8). On the other hand, the above-mentioned p-aminophenylglycoside is allowed to react with maleic anhydride in anhydrous tetrahydrofran to give an unsaturated amido-carboxylic acid compound (Embodiment 7). Next, the said amido-carboxylic acid compound is allowed to react with sodium methoxide in anhydrous methanol, then is neutralized by Dowex 50w ($H^+$) to give deacetylated substance (Embodiment 9).

Because the sialic acid derivatives of this invention shown by the aforementioned formula [I] contain the

active ester group in the molecules, they exhibit high reactivity to other compounds containing functional groups that can react with ester groups, such as amino compounds. That is, the sialic acid derivative containing active ester groups of this invention is an extremely useful compound as raw material or intermediate to synthesize various sialic acid derivatives.

Other sialic acid derivative of this invention is that expressed by the formula [II] as follows:

[II]

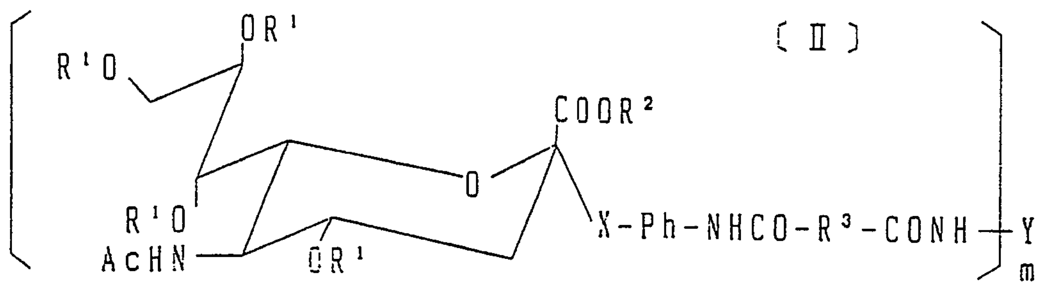

where $R^1$ denotes hydrogen or acetyl group, $R^2$ hydrogen or lower alkyl group, $R^3$ $C_2H_4$, $C_3H_6$ or $C_2H_2$, Ac acetyl group, m 1-60, Ph phenyl group, X oxygen or sulfur, and Y the residue left from removing m number of amino group from amino compounds. The amino compounds include amine of lower class and amino acids.

The sialic acid derivative can be prepared from amino compounds and sialic acid derivatives containing aforementioned active ester group by the use of active ester process and mixed acid anhydride process.

The active ester process produces N-oxysuccinimide ester substance by allowing the said amido-carboxylic acid compound with DSC (N,N′-disuccinimidyl carbonate) in anhydrous acetonitrile (Embodiment 10). In this event, adding anhydrous pyridine at more than equivalent mole ratio causes the N-oxysuccinimide ester substance to transfer to the p-succinimidophenylglycoside substance of intramolecular ring closure (Embodiment 11). The amido-carboxylic acid compound is allowed to react in anhydrous tetrahydrofran in the presence of WSC (1-ethyl-3-(3-dimethylaminopropyl)-carbodiimide) of the condensing agent to give p-nitrophenyl ester substance (Embodiment 12). The said ester substance is not isolated and is allowed to react by adding amino acid methyl ester in the solution to give amide substance, the sialic acid derivative of this invention (Embodiment 13-1).

On the other hand, in the mixed acid anhydride process, the said amido-carboxylic compound is allowed to react with isobutylchloroformate in anhydrous tetrahydrofran to give a mixed acid anhydride, then, the mixed acid anhydride is allowed to react with amino acid methyl ester to give the amide substance of this invention (Embodiment 13-2). By the way, the use of this mixed acid anhydride process can produce the amide substance from deacetyl substance of the said amido-carboxylic acid compounds under the similar reaction conditions (Embodiment 15).

For other processes, there is a method to produce a peracetylated substance by allowing the said amide substance to react with acetic anhydride in anhydrous pyridine (Embodiment 13-3). As reaction species other than amino acid ester, hydrazine is allowed to react with the said N-hydroxysuccinimide ester isolated or in the solution in anhydrous acetonitrile to give acid hydrazide (Embodiment 14).

The sialic acid derivatives of this invention expressed by the above-mentioned formula [II] are compounds consisting of sialic acid derivative expressed by the aforementioned formula [I] and amino compounds. For example, when amino acid is administered to animals or human bodies as nutrient, or when insulin, growth hormone, interferon, and immunogen are administered as medicine, it is predicted that administration of these medicines as the sialic acid derivative expressed with the said formula [II] will prevent or delay biological reactions of biologically active substance by the presence of sialic acid. This will produce good effects of increasing durability of biologically active substance in the body or displaying desired medicinal effects with a small amount of administration. That is, the sialic acid derivative expressed by the said formula [I] is an extremely useful compound as extender of biological half-life of various biologically active substances.

## DETAILED DESCRIPTION OF THE PREFERRED EMBODIMENTS

Referring now to application examples, embodiments of the sialic acid derivative according to the

present invention will be described in detail hereafter. However, the present invention shall not be limited by any of the detail of these embodiments.

[Embodiment 1]

Synthesis of methyl (4-nitrophenyl 5-acetamido-4, 7, 8, 9-tetra-O-acetyl-3, 5-dideoxy-α-D-glycero-D-galacto-2-nonulopyranosido)onate:

(1) N-acetylneuraminic acid was allowed to react in ethanol in the presence of Dowex 50w ($H^+$) at the room temperature for six hours to give methyl ester substance (yield = 80 %).

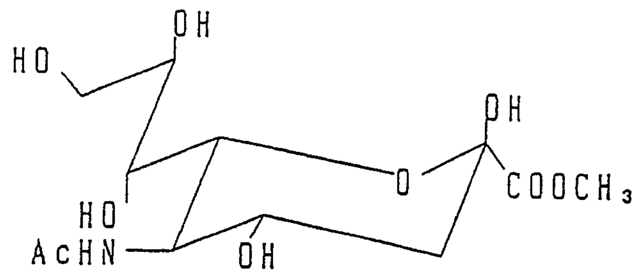

(2) After the said methyl ester substance was allowed to react with excess acetyl chloride for one day, ethanol was added with cooling (-30 °C), and it was allowed to stand for 10 days to have chlor substance (yield = 83 %)

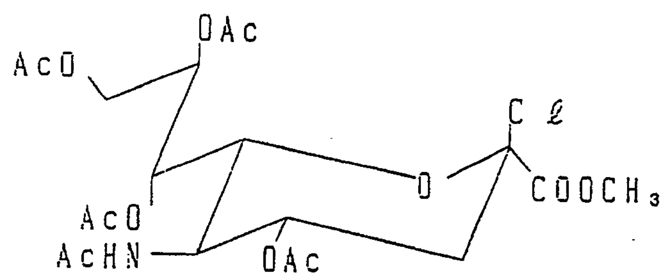

(3) 10.26 g of anhydrous sodium salt of p-nitrophenol and 6.0 g of the chlor substance obtained in Section 2 (methyl 5-acetamide-4, 7, 8, 9-tetra-O-acetyl-2-chloro-3,5-dideoxy- -D-glycero-D-galacto-nonulopyranosonate) were dissolved in 120 ml of anhydrous dimethylformamide, and were allowed to react with stirring for 24 hours under the moisture-proof condition.

Then, the solvent was removed from the solution under the depressurizing condition, xylene was added, and solvent was repeatedly removed. Ethyl acetate was added to the residue obtained and stirred, and the residue was extracted thoroughly with ethyl acetate. The solvent was removed from the extract liquid and the residue was applied with silica gel column chromatography (Wakogel C-300). At first, p-nitrophenol was eluted from the residue with ether, and after removal, it was eluted with ethyl acetate to give the fractional solution containing the object. The solvent was removed from the fractional solution and oily substance (crude yield point = 6.87 g, crude yield = 95.4 %, melting point = 95 - 98 °C) was obtained.

TLC: Rf = 0.41 (Kieselgel 60 $F_{254}$, Merck product, acetate)
Rf = 0.44 (Kieselgel 60 $F_{254}$, Merck product, $CHCl_3$ /MeOH = 20/1

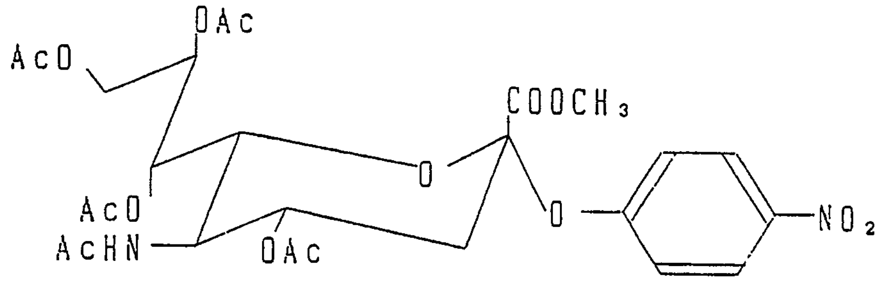

(Reference)

1) In Volker Eschenfelder and Reinhard Brossmer, Carbohydrate Research 162 (1987) 294-297, the aforementioned synthesis method is described, but the yield is as poor as 57 % (melting point = 104 -108° (dec.) (ether/hexane).

[Physical properties of the product]

$$^{1}H\text{-}NMR^{ppm}_{500MHz}\ (CDCl_3,\ TMS)$$

1 .932 (3H, s, $-NHCOCH_3$),
2.055;2.061;2.119;2.193 (all 3H, all s, $-OCOCH_3$ X 4),
2.304 (1H, t, J = 12.8Hz, $H_{3ax}$),
2.744 (1H, dd, J = 13.2Hz, 4.8Hz, $H_{3eq}$),
3.663 (3H, s, $2-COOCH_3$),
4.986 (1H, ddd, J = 12.1, 10.3, 4.8Hz, H-4),
7.153 (2H, d, J = 9.2Hz, phenyl-H),
8.189 (2H, d, J = 9.2Hz, phenyl-H).
$IR\nu^{KBr}_{max}$ $cm^{-1}$ : 1740, 1660, 1520, 1340, 1220,

[Embodiment 2]

Synthesis of methyl (4-nitrophenyl 5-acetamido-4, 7, 8, 9-tetra-O -acetyl-2, 3, 5-trideoxy-2-thio-α-D-glycero-D-galacto-2-nonulopyranosido)onate:

Anhydrous sodium salt of p-nitrothiohenol prepared from 19.6 ml of methanol solution of 1 .67 g of p-nitrothiophenol, 0.5 mol of sodium methoxido, and 1.0 g of chlor substance obtained in the aforementioned Embodiment 1 (2) (methyl 5-acetamide-4, 7, 8, 9-tetra-O-acetyl-2-chloro-3, 5-dideoxy-β-D-glycero-D-galacto-2-nonulopyranosonate) were dissolved in 15 ml of anhydrous dimethylformamide and allowed to react with stirring for 6 hours at room temperature under moisture-proof and nitrogen-substituting atmosphere. Then, after solvent was removed under the depressurizing condition, xylene was added and solvent was repeatedly removed. The residue obtained was applied with silica gel column chromatography (Wakogel C-300, ethyl acetate) twice and solvent was again removed from the fractional solution, and finally 0.52 g white powder was obtained (yield = 42.4 %, melting point = 94 - 96 °C).
TLC: Rf = 0.44 (Kieselgel 60 $F_{254}$, Merck product, ethyl acetate)

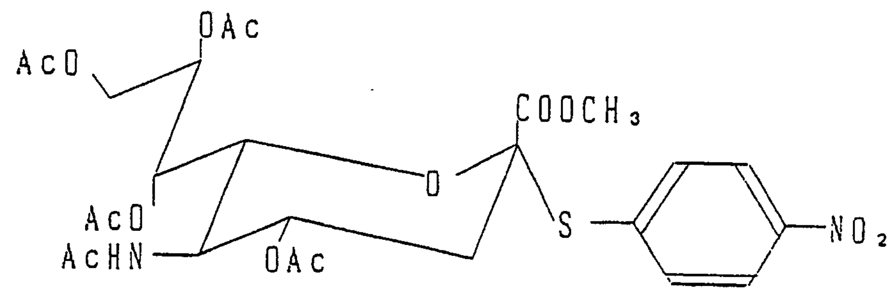

[Physical properties of the product]

$C_{26}H_{32}O_{14}N_2S$
FAB-MS m/z : 629 ($M^+$ + 1)

$^1H\text{-NMR}^{ppm}_{500MHz}$ ($CDCl_3$, TMS)

1.891 (3H, s, $-NHCOCH_3$),
2.041;2.061;2.063;2.165 (all 3H, all s,$-OCOCH_3$ X 4),
2.869 (1H, dd, J = 12.8, 4.8Hz, $H_{3eq}$),
3.611 (3H, s, 2-$COOCH_3$),
4.883 (1H, ddd, J = 12.1, 10.3, 4.8Hz, H-4),
7.655 (2H, d, J = 8.8Hz, phenyl-H),
8.192 (2H, d, J = 8.8Hz, phenyl-H).
IR$\nu^{KBr}_{max}$ $cm^{-1}$ : 3250, 1750, 1650, 1550, 1520, 1350,

[Embodiment 3]

Synthesis of methyl (4-aminophenyl 5-acetamido-4, 7, 8, 9-tetra-O-acetyl-3, 5-dideoxy-α-D-glycero-D-galacto-2-nonulopyranosido)onate:

6.87 g of methyl (4-nitrophenyl 5-acetamido-4, 7, 8, 9-tetra-O-acetyl-3, 5-dideoxy-α-D-glycero-D-galacto-2-nonulopyranosido)onate) obtained in the aforementioned Embodiment 1 (3) were dissolved in 50 ml of methanol. After a slight amount of 5 % palladium/carbon was added with a spatula, it was reduced with hydrogen with stirring at room temperature. Then after allowing to react for 2 days, reagents were removed by filtering. Solvent was removed from the filtrate and 4.91 g of oily substance (yield = 71.63 %) was obtained. It was further purified by the use of silica gel column chromatography (Wakogel C-300, $CHCl_3$/MeOH-40/1) and white powdery crystal (melting point: 95 - 99 °C) was obtained as TLC one spot purified product as shown below.

TLC: Rf = 0.29 (Kieselgel 60 $F_{254}$, Merck product, ethyl acetate)
Rf = 0.35 (Kieselgel 60 $F_{254}$, Merck product, $CHCl_3$ /MeOH = 20/1)

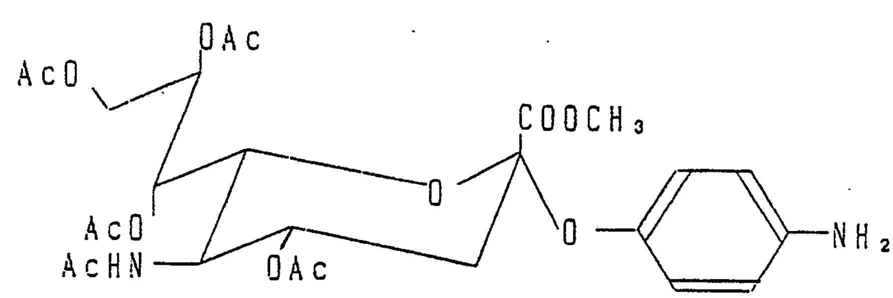

[Physical properties of the product]

$C_{26}H_{34}O_{13}N_2$
FAB-MS m/z : 583 ($M^+$ + 1)

$^1$H-NMR$^{ppm}_{500MHz}$ ($CDCl_3$, TMS)

1.896 (3H, s, $-NHCOCH_3$),
2.028;2.055;2.115;2.139 (all 3H, all s,$-OCOCH_3$ X 4),
2.676 (1 H, dd, J = 12.8, 4.8Hz, $H_{3eq}$),
3.671 (3H, s, 2-$COOCH_3$),
4.936 (1H, J = 12.1, 10.3, 4.8Hz, H-4),
6.569 (2H, d, J = 8.8Hz, phenyl-H),
6.886 (2H, d, J = 8.8Hz, phenyl-H).
IR$\nu^{KBr}_{max}$ $cm^{-1}$ : 3450, 3370, 1740, 1660, 1540,

[Embodiment 4]

Synthesis of methyl (4-dimethylaminophenyl 5-acetamido-4, 7, 8, 9-tetra-O-acetyl-2, 3, 5-trideoxy-2-thio-α-D-glycero-D-galacto-2-nonulopyranosido)onate:

0.25 g of [methyl (4-nitrophenyl 5-acetamido-4, 7, 8, 9-tetra-O-acetyl-2, 3, 5-trideoxy-2-thio-α-D-glycero-D-galacto-2-nonulopyranoside)onate) obtained in the aforementioned Embodiment 2 were dissolved in 10 ml of methanol. After a slight amount of 5 % palladium/carbon was added with a spatula, it was reduced with hydrogen with stirring at room temperature for one day. Then reagents were removed by filtering. The solvent was removed from the filtrate and the residue was applied with silica gel column chromatography (Wakogel C-300, $CHCl_3$ /MeOH = 40/1). Solvent was removed from the fractional solution containing the object and 100 mg of white powdery crystal was obtained (yield = 40 %, melting point = 83 -85 °C).
TLC: Rf = 0.50 (Kieselgel 60 $F_{254}$, Merck product, chloroform/methanol = 20/1)

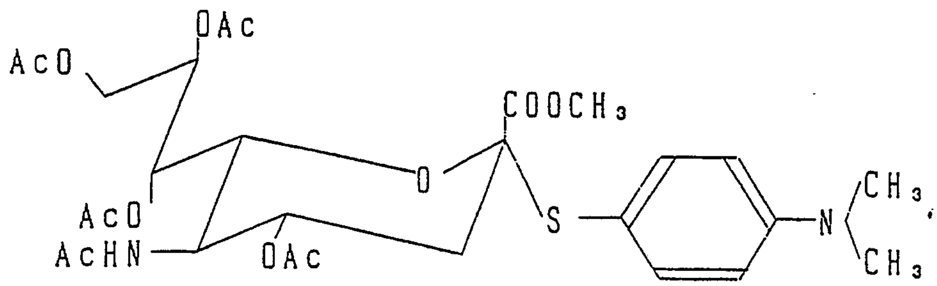

[Physical properties of the product]

$C_{28}H_{38}O_{12}N_2S$
FAB-MS m/z : 627 ($M^+$ + 1)

$^1$H-NMR$^{ppm}_{500MHz}$ ($CDC_{13}$, TMS)

1.853 (3H,.s, $-NHCOCH_3$),
1.970 (1H, t, J = 12.8Hz, $H_{3ax}$),
2.011;2.054;2.133 (3H;6H;3H, all s, $-OCOCH_3$ X 4),
2.742 (1H, dd, J = 12.8, 4.8Hz, $H_{3eq}$),

2.987 (6H, s, 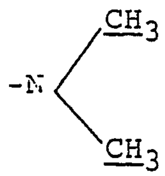 )

3.636 (3H, s, 2-$COOCH_3$),
4.829 (1 H, ddd, J = 11.7, 10.3, 4.8Hz, H-4),
6.614 (2H, d, J = 8.8Hz, phenyl-H),
7.330 (2H, d, J = 8.8Hz, phenyl-H).
$IR\nu^{KBr}_{max}$ $cm^{-1}$ : 3360, 1740,

[Embodiment 5]

Synthesis of methyl (4-aminophenyl 5-acetamido-4, 7, 8, 9-tetra-O-acetyl-2, 3, 5-trideoxy-2-thio-α-D-glycero-D-galacto-2-nonulopyranosido)onate:

0.10 g of methyl (4-nitrophenyl 5-acetamido-4, 7, 8, 9-tetra-O-acetyl-2, 3, 5-trideoxy-2-thio-α-D-glycero-D-galacto-2-nonulopyranosido)onate) obtained in the aforementioned Embodiment 2 were dissolved in 5 ml of acetic acid. After a slight amount of 5 % palladium/carbon was added with a spatula, it was reduced with hydrogen with stirring at room temperature. Then reagents were removed by filtering. Solvent was removed from the filtrate and the residue was applied with silica gel column chromatography (Wakogel C-300, $CHCl_3$ /MeOH = 40/1 ). In that event, the solution of the residue was neutralized with triethylamine, then developed. Solvent was removed from the fractional solvent containing the object and 67 mg of white powdery crystal was obtained (yield = 70 %, melting point = 100 - 102 °C).
TLC: Rf = 0.29 (Kieselgel 60 $F_{254}$, Merck product, chloroform/methanol = 20/1)

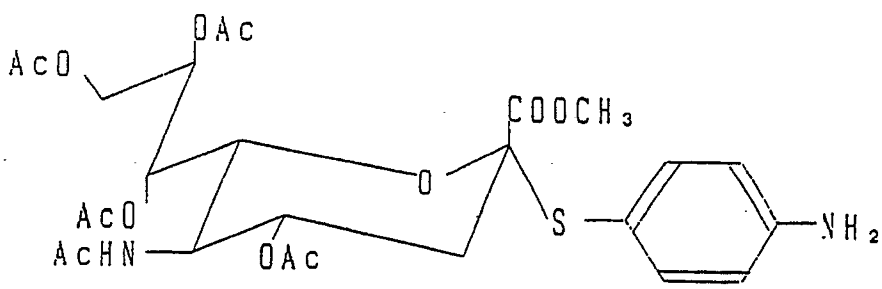

[Physical properties of the product]

$C_{26}H_{34}O_{12}N_2S$
FAB-MS m/z : 599 ($M^+$ + 1)

$^1H\text{-}NMR^{ppm}_{500MHz}$ ($CDCl_3$, TMS)

1.858 (3H, s, -$NHCOCH_3$),
1.978 (1H, t, J = 12.5Hz, $H_{3ax}$),
2.016;2.054;2.060 2.138 (all 3H, all s, -$OCOCH_3$ X 4),
4.144 (1H, dd, J = 12.8, 4.8Hz, $H_{3eq}$),
3.618 (3H, s, 2-$COOCH_3$),
4.833 (1H, ddd, J = 11.7, 10.3, 4.8Hz, H-4),
6.603 (1H, d, J = 8.8Hz, phenyl-H),
7.266 (1H, d, J = 8.8Hz, phenyl-H),

$IR\nu_{max}^{KBr}$ $cm^{-1}$ : 3470, 3380, 1740,

[Embodiment 6]

Synthesis of 4′-[(methyl 5-acetamido-4, 7, 8, 9-tetra-O-acetyl-3, 5-dideoxy-α-D-glycero-D-galacto-2-nonylopyranosylonate) oxy] succinanilic acid:

4.91 g of [methyl (4-aminophenyl 5-acetamido-4, 7, 8, 9-tetra-O-acetyl-3, 5-dideoxy-α-D-glycero-D-galacto-2-nonulopyranosido)onate] obtained in the aforementioned Embodiment 3 and 1.26 g of succinic anhydride were dissolved in 100 ml of anhydrous tetrahydrofuran and allowed to react for one day with stirring at room temperature. After the disappearance of raw materials was confirmed with a TLC, reaction solvent was removed under the depressurizing condition, and the residue was applied with gel filtration chromatography (LH-20, MeOH) and the fractional solution not containing succinic anhydride was obtained. The residue obtained by removing solvent from the fractional solution was recrystallized with acetate, and 4.81 g of the object was obtained (total upto the third crystal, yield = 83.7 %, melting point = 130 - 131 °C).

TLC: Rf = 0.13 (Kieselgel 60 $F_{254}$, Merck product, $CHCl_3$ /MeOH = 20/1)

Rf = 0.42 (Kieselgel 60 $F_{254}$, Merck product, $CHCl_3$ /MeOH = 10/3)

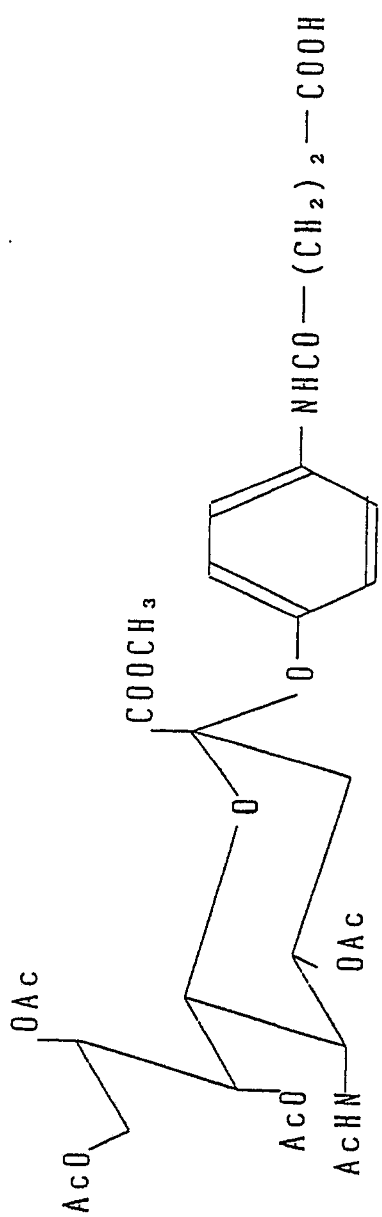

[Physical properties of the product]

$C_{30}H_{38}O_{16}N_2$
FAB-MS m/z : 683 ($M^+$ + 1)

$^{1}H\text{-}NMR^{ppm}_{500MHz}$ ($CDCl_3$, TMS)

1.890 (3H, s, $-NHCOCH_3$),
2.041;2.051;2.121 2.126 (all 3H, all s, $-OCOCH_3$ X 4),
2.186 (1H, t, J=12.5Hz, $H_{3ax}$),
2.655 - 2.710 (3H, m, $-CH_2CH_2-$ + $H_{3eq}$)),
2.777 (3H, t, J=6.6Hz, $-CH_2CH_2-$)
3.643 (3H, s, $-COOCH_3$),
4.964 (2H, ddd, J=12.1, 10.3, 4.8Hz, H-4),

7.011 (2H, d, J = 9.2Hz, phenyl-H),
7.418 (2H, d, J = 9.2Hz, phenyl-H).
IR$\nu_{max}^{KBr}$ $cm^{-1}$ : 3350, 1740, 1660, 1540,

[Embodiment 7]

Synthesis of 4′-[(methyl (5-acetamido-4, 7, 8, 9-tetra-O-acetyl-3, 5-dideoxy-α-D-glycero-D-galacto-2-non-ulopyranosylonate)oxy] maleanilic acid:

100 g of [methyl (4-aminophenyl 5-acetamido-4, 7, 8, 9-tetra-O-acetyl-3, 5-dideoxy-α-D-glycero-D-galacto-2-nonulopyranosido)onate] obtained in the aforementioned Embodiment 3 and 24 mg of maleic anhydride were dissolved in 3 ml of anhydrous tetrahydrofuran and stirred at room temperature, then reactions took place immediately. After the disappearance of raw materials was confirmed with a TLC, reaction solvent was removed under the depressurizing condition. The residue was applied with gel filtration chromatography (LH-20, MeOH) and the fractional solution containing the object was obtained. From this fractional solution, solvent was removed and 74.8 mg of pale yellow powdery crystal was obtained (yield = 67.4 %, melting point = 121 - 123 °C).
TLC: Rf = 0.33 (Kieselgel 60 $F_{254}$, Merck product, $CHCl_3$ /MeO = 10/3)

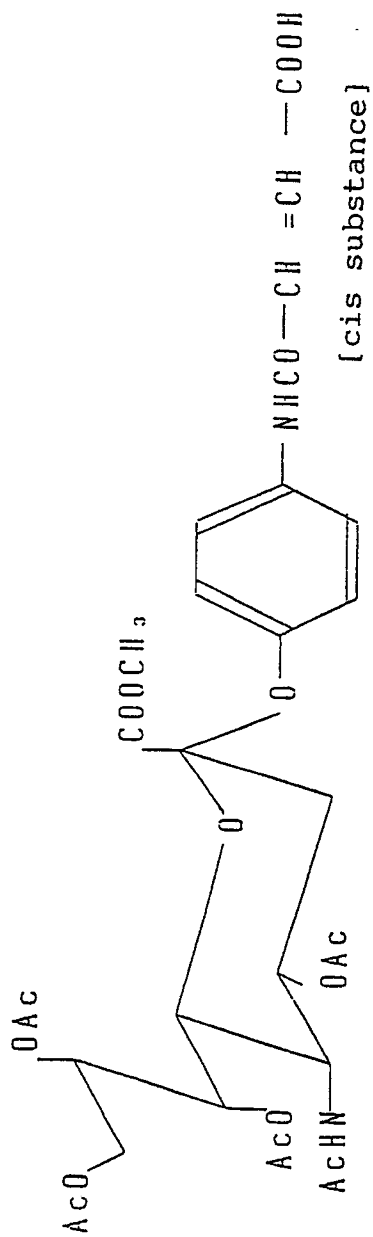

[cis substance]

[Physical properties of the product]

$C_{30}H_{36}O_{16}N_2$
FAB-MS m/z : 681 (M+ + 1)

$^{1}$H-NMR$^{ppm}_{500MHz}$ ($CDCl_3$, TMS)

1.929 (3H, s, $-NHCOCH_3$),
2.047;2.095;2.140 2.145 (all 3H, all s, $-OCOCH_3$ X 4),
2.243 (1H, t, J = 12.8Hz, $H_{3ax}$),
2.715 (1H, dd, J = 12.8, 4.4Hz, $H_{3eq}$),
3.654 (3H, s, $2-COOCH_3$),
4.955 (1H, ddd, J = 12.1, 10.6, 4.4Hz, H-4),

6.455 (1H, d, J = 12.8Hz, olefin-H),
6.499 (1H, d, J = 12.8Hz, olefin-H),
7.048 (2H, d, J = 8.8Hz, phenyl-H),
7.543 (2H, d, J = 8.8Hz, phenyl-H),
IR$\nu_{max}^{KBr}$ $cm^{-1}$ : 3350, 1750, 1770, 1550,

[Embodiment 8]

Synthesis of 4′-[methyl (5-acetamido-4, 7, 8, 9-tetra-O-acetyl-3, 5-dideoxy-α-D-glycero-D-galacto-2-nonulopyranosylonate) thio] succinanilic acid:

0.1 g of [methyl (4-aminophenyl 5-acetamide-4, 7, 8, 9-tetra-O-acetyl-3, 5-dideoxy-α-D-glycero-D-galacto-2-nonulopyranosido)onate] obtained in the aforementioned Embodiment 5 and 20 mg of succinic anhydride were dissolved in 3 ml of anhydrous tetrahydrofuran and were allowed to react with stirring for one day at room temperature. After the disappearance of raw materials was confirmed with a TLC, reaction solvent was removed under the depressurizing condition. The residue was applied with gel filtration chromatography (LH-20, MeOH) and fractional solution containing the object was obtained. From this fractional solution, solvent was removed and 9.4 mg of white powdery crystal was obtained (yield = 82 %, melting point = 119 - 121 °C).
TLC: Rf = 0.40 (Kieselgel 60 $F_{254}$, Merck product, $CHCl_3$ /MeOH = 10/3)

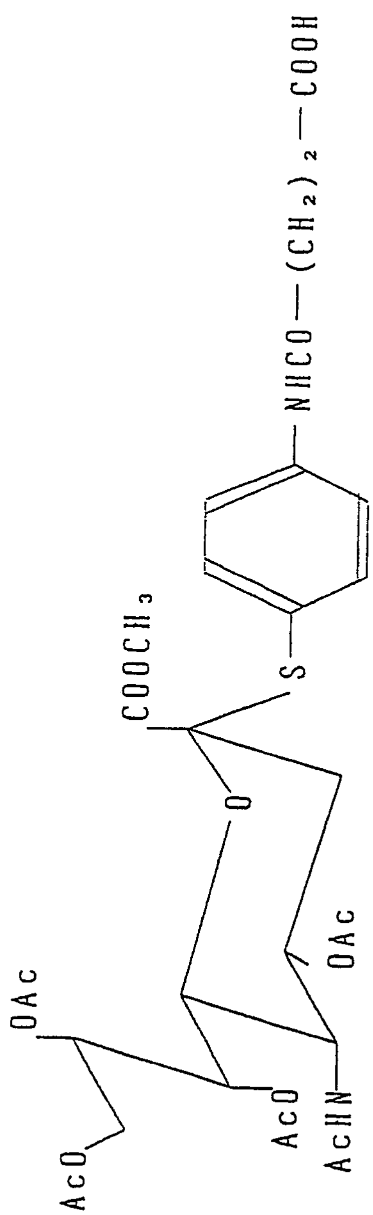

[Physical properties of the product]

$C_{30}H_{38}O_{15}N_2S$
FAB-MS m/z : 699 ($M^+$ + 1)
$^1$H-NMR 500MHz ($CDCl_3$, TMS)
1.844 (3H, s, $-NHCOCH_3$),
2.019;2.038;2.059;2.127 (all 3H, all s, $-OCOCH_3$ X 4),
2.65 - 2.80(5H, m, $-CH_2CH_2-$ + $H_{3eq}$),
3.585 (3H, s, 2-$COOCH_3$),
4.837 (1H, ddd, J = 11.4, 10.3, 4.8Hz, H-4),
7.414 (2H, d, J = 8.4Hz, phenyl-H),
7.534 (2H, d, J = 8.4Hz, phenyl-H),
IR$\nu_{max}^{KBr}$ $cm^{-1}$ : 3340, 1740, 1220,

[Embodiment 9]

Synthesis of 4′-[(methyl 5-acetamido-3, 5-dideoxy-α-D-glycero-D-galacto-2-nonulopyranosylonate)oxy] succinanilic acid and its sodium salts:

222 mg of 4′-[(methyl 5-acetamido-4, 7, 8, 9-tetra-O-acetyl-3, 5-dideoxy-α-D-glycero-D-galacto-2-nonulopyranosylonate)oxy] succinanilic acid obtained in the aforementioned Embodiment 6 were dissolved in 20 ml of anhydrous methanol and 386 mg of 28 % methanol solution of sodium methoxide were added at room temperature and allowed to react with stirring for two hours. Then, while cooling, 0.8 g of Dowex 50w ($H^+$) were added and stirred to be made slightly acidic to pH 4. The ion exchange resin was removed by filtering and solvent was removed from the filtrate liquid obtained, and amorphous substance was obtained. The residue obtained was applied with $C_{18}$-column chromatography (YMC.GEL ODS 60 Å 60/200 mesh). The residue was eluted first with water, then with methanol, and water was added to the methanol fractional solution containing the object, freeze-dryed to give 100 mg of white powdery crystal of free-acid type carboxylic acid (yield = 60 %, melting point = 132 - 135 °C). The sodium salts were isolated with the similar operation when the amount of the aforementioned Dowex 50w (H+) was less than a half.
TLC: Rf = 0.40 (Kieselgel 60 $F_{254}$, Merck product, $CHCl_3$ /MeOH = 6/3/0.5)

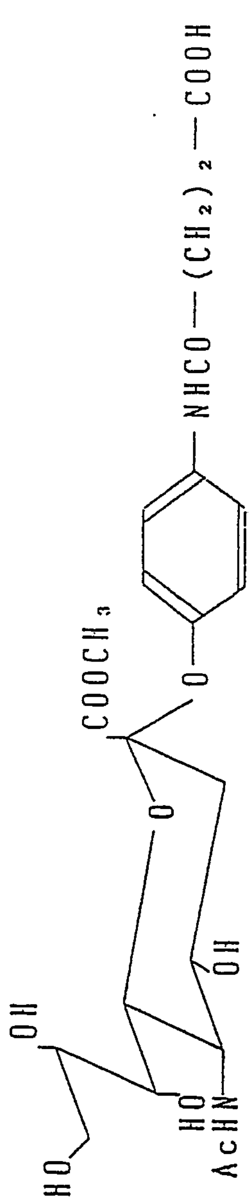

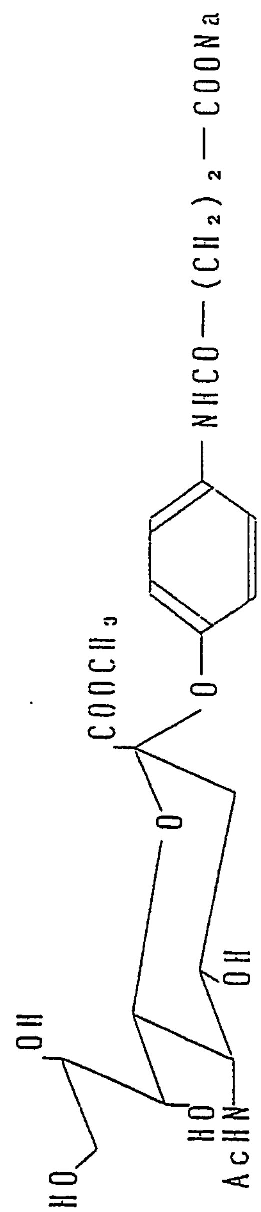

[Physical properties of the product (COOH substance)]

Element analysis $C_{22}H_{30}O_{12}N_2$
FAB-MS m/z : 515 ($M^+$ + 1)

$$^1H\text{-NMR}^{ppm}_{500MHz}(D2O,\ TSP)$$

2.030 (1H, t, J=12.5Hz, $H_{3ax}$),
2.039 (3H, s, $-NHCOCH_3$),
2.67 - 2.69(4H, m, $-\overline{CH_2}-CH_2-$),
2.880 (1H, dd, J=12.8, 4.8Hz, $H_{3eq}$),
3.748 (3H, s, $2-COOCH_3$),
7.150 (1H, d, J=8.8Hz, phenyl-H),
7.386 (1H, d, J=8.8Hz, phenyl-H),
IR$\nu^{KBr}_{max}$ $cm^{-1}$ : 3400, 1730, 1660, 1550,

[Embodiment 10]

Synthesis of [4′-(3- (N-succinimidyloxycarbonyl)propionamido) phenyl 5-acetamido-4, 7, 8, 9-tetra-O-acetyl-3, 5-dideoxy-α-D-glycero-D-galacto-2-nonulopyranosido]onate:

100 mg of 4′-[(methyl 5-acetamido-4, 7, 8, 9-tetra-O-acetyl-3, 5-dideoxy-α-D-glycero-D-galacto-2-nonulopyranosylonate)oxy] succinanilic acid obtained in the aforementioned Embodiment 6 and 38 mg of N, N′-disuccinimidyl carbonate were dissolved in 10 ml of anhydrous acetonitrile and 11.7 μl of anhydrous pyridine were added and allowed to react with stirring at room temperature.

After disappearance of raw material was confirmed with a TLC, reaction solvent was removed under the depressurizing condition. The residue obtained was applied with silica gel column chromatography (Wakogel C=300, toluene/acetone = 1/1). The residue of the obtained fractional solution is further applied with a gel filtration chromatography (LH-20, toluene/acetone = 1/1) and 91 mg of white powdery crystal was obtained (yield = 80 %, melting point = 115 - 117 °C).
TLC: Rf = 0.27 (Kieselgel 60 $F_{254}$, Merck product, toluene/acetone = 1/1)

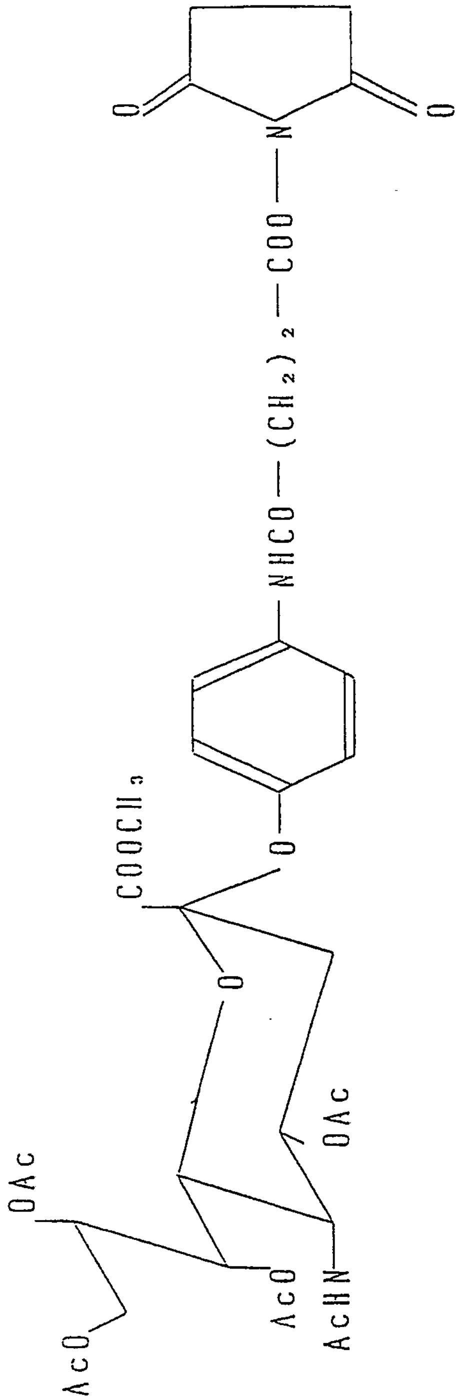

[Physical properties of the product]

$C_{34}H_{41}O_{18}N_3$
FAB-MS m/z : 780 ($M^+$ + 1)

$$^1H\text{-}NMR^{ppm}_{500MHz}(CDCl_3,\ TMS)$$

1.902 (3H, s, $-NHCOCH_3$),
2.036;2.051;2.118;2.133 (all 3H, all s, $-OCOCH_3$ X 4),

2.183 (1H, t, J=12.8Hz, $H_{3ax}$),
2.699 (1H, dd, J=12.8, 4.8Hz, $H_{3eq}$),
2.759 (2H, t, J=7.0Hz, $-CH_2-CH_2-$),
3.056 (2H, t, J=7.0Hz, $-CH_2-CH_2-$),
2.844 (4H, s, H of succinimidyl group),
3.658 (3H, t, $2-COOCH_3$),
4.944 (1H, ddd, J=12.5, 10.3, 4.8Hz, H-4),
7.022 (2H, d, J=8Hz, phenyl-H),
7.413 (1H, d, J=8Hz, phenyl-H),
IR$\nu_{max}^{KBr}$ $cm^{-1}$ : 3360, 1740, 1670, 1540,

[Embodiment 11]

Synthesis of [4-succinimidophenyl 5-acetamido-4, 7, 8, 9-tetra-O-acetyl-3, 5-dideoxy-α-D-glycero-D-galacto-2-nonulopyranosido]onate:

120 mg of 4′-[(methyl 5-acetamido-4, 7, 8, 9-tetra-O-acetyl-3, 5-dideoxy-α-D-glycero-D-galacto-2-nonulopyranosylonate)oxy] succinanilic acid obtained in the aforementioned Embodiment 6 and 247 mg of N, N′-disuccinimidyl carbonate and 340 mg of anhydrous pyridine were dissolved in 20 ml of anhydrous acetonitrile and allowed to react at room temperature for one day. After reaction solvent was removed, the residue obtained was applied with silica gel column chromatography (C=300, toluene/acetone = 1/1). The solvent was removed from the obtained fractional solution containing the object and white powdery crystal was obtained. Then it was further applied with a gel filtration chromatography (LH-20, ethyl acetate) and 24 mg of white powdery crystal was obtained (yield = 20 %, melting point = 110 - 114 °C).
TLC: Rf = 0.35 (Kieselgel 60 $F_{254}$, Merck product, toluene/acetone = 1/1)
Rf = 0.26 (Kieselgel 60 $F_{254}$, Merck product, ethyl acetate)

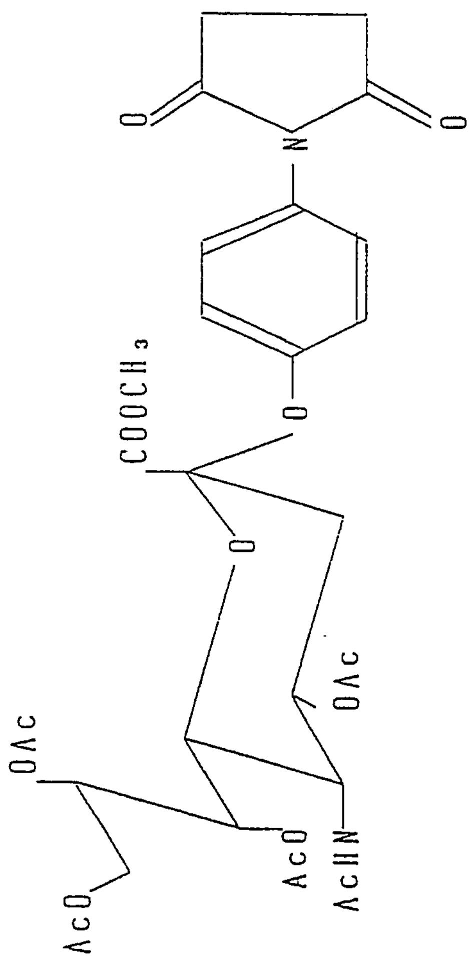

[Physical properties of the product]

$C_{30}H_{36}O_{15}N_2$
FAB-MS m/z : 665 ($M^+$ + 1)

$^1H\text{-NMR}\,{}^{ppm}_{500MHz}$ 500MHz ($CDCl_3$, TMS)

1.913 (3H, s, $-NHCOCH_3$),
2.047;2.049;2.121;2.153 (all 3H, all s, $-OCOCH_3$ X 4),
2.250 (1H, t, J = 12.8Hz, $H_{3ax}$),
2.711 (1H, dd, J = 12.8, 4.8Hz, $H_{3eq}$),
2.875 (4H, s, H of succinimido group),
3.687 (3H, s, $2-COOCH_3$),
4.971 (1H, ddd, J = 12.1, 10.6, 4.8Hz, H-4),
7.140 (2H, d, J = 8.8Hz, phenyl-H),
7.203 (1H, d, J = 8.8Hz, phenyl-H),
$IR\nu^{KBr}_{max}$ $cm^{-1}$ : 3460, 3360, 1750, 1710,

[Embodiment 12]

Synthesis of [4-(3-p-nitrophenyloxycarbonylpropionamide) phenyl 5-acetamido-4, 7, 8, 9-tetra-O-acetyl-3, 5-dideoxy-α-D-glycero-D-galacto-2-nonulopyranosido]onate:

52 mg of 4′-[(methyl 5-acetamido-4, 7, 8, 9-tetra-O-acetyl-3, 5-dideoxy-α-D-glycero-D-galacto-2-nonulopyranosylonato)oxy] succinanilic acid obtained in the aforementioned Embodiment 6, 24.1 mg of p-nitrophenol, and 14.5 mg of WSC (1-ethyl-3-(3-dimethylaminopropyl)-carbodiimide hydrochloride] were dissolved in 1 ml of tetrahydrofuran and allowed to react at 0 °C with stirring. After disappearance of raw material was confirmed with a TLC, reaction solvent was removed and the residue obtained was applied with silica gel column chromatography (Wakogel C=300, toluene/acetone = 1/1) to give the fractional solution containing the object. The solvent was removed from the obtained fractional solution and 20 mg of white powedry crystal was obtained (yield = 32.7 %, melting point = 106 - 108 °C).

TLC: Rf = 0.38 (Kieselgel 60 $F_{254}$, Merck product, ethyl acetate)

Rf = 0.42 (Kieselgel 60 $F_{254}$, Merck product, toluene/acetone = 1/1).

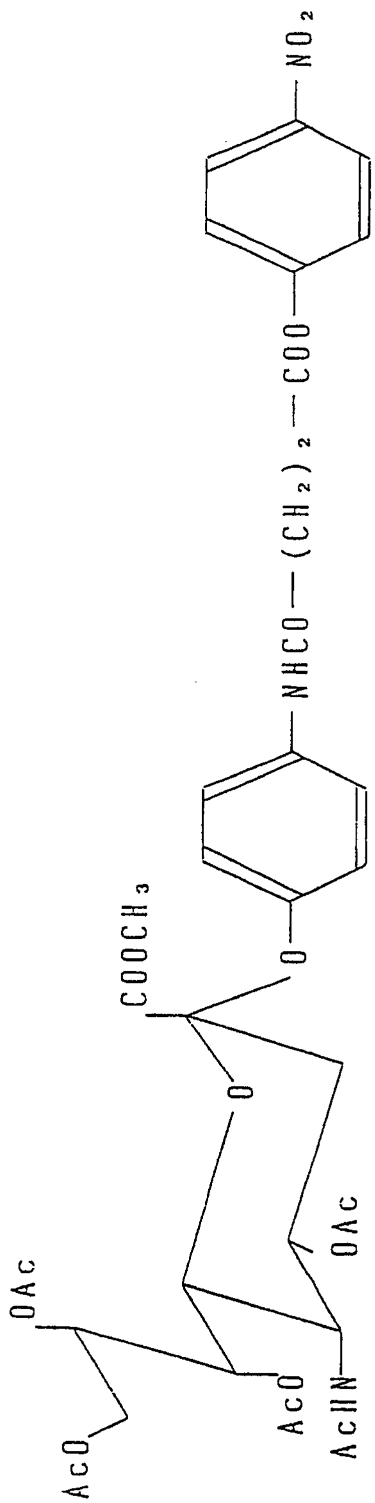

[Physical properties of the product]

$C_{36}H_{41}O_{18}N_3$
FAB-MS m/z : 804 ($M^+$ + 1)

$^1H\text{-}NMR^{ppm}_{500MHz}(CDCl_3, TMS)$

1.908 (3H, s, $-NHCOCH_3$),
2.038;2.047;2.126;2.132 (all 3H, all s, $-OCOCH_3$ X 4),
2.191 (1H, t, J=1 2.8Hz, $H_{3ax}$),
2.708 (1H, dd, J=12.8, 4.4Hz, $H_{3eq}$),
2.779 (2H, t, J=6.6Hz, $-CH_2-\underline{CH_2}-$),
3.019 (2H, t, J=6.6Hz, $-\overline{CH_2}-\underline{CH_2}-$),
3.656 (3H, t, $2-COOCH_3$),
4.947 (1H, ddd, J=12.1, 10.3, 4.4Hz, H-4),
7.031 (2H, d, J=8.8Hz, H of nitrophenyl group),
7.313 (2H, d, J=8.8Hz, aromatic ring-H of anilic acid),
7.409 (2H, d, J=8.8Hz, aromatic ring-H of anilic acid),
8.265 (2H, d, J=8.8Hz, nitrophenyl group).
$IR\nu^{KBr}_{max}$ $cm^{-1}$ : 3350, 1740, 1660, 1520, 1340,

[Embodiment 13]

Synthesis of N-(4′-[(methyl 5-acetamido-4, 7, 8, 9-tetra-O-acetyl-3, 5-dideoxy-α-D-glycero-D-galacto-2-nonulopyranosylonate)oxy] succinaniloyl)glycine methyl ester:

[13-1] The first method (active ester process)

100 mg of 4′-[(methyl 5-acetamido-4, 7, 8, 9-tetra-O-acetyl-3, 5-dideoxy-α-D-glycero-D-galacto-2-non-ulopyranosylonate) oxy] succinanilic acid obtained in the aforementioned Embodiment 6, 38 mg of N,N′-disuccinimidyl carbonate, and 11.7 µl of anhydrous pyridine were dissolved in 10 ml of anhydrous acetonitrile and allowed to react at room temperature for 2 hours. After disappearance of raw material was confirmed with a TLC, acetontrile solution consisting of 18.4 mg of glycine methyl ester hydrochloride and 20.4 µl of triethylamine was added to the mixture of the above-mentioned active ester, and the solution was allowed to react with stirring for one day. After disappearance of active ester with a TLC, reaction solvent was removed and the residue obtained was applied with silica gel column chromatography (Wakogel C = 300, $CHCl_3$/MeOH = 20/1), then further with gel filtration chromatography (LH-20, MeOH) to give fractional solution containing the object. Solvent was removed from the obtained fractional solution and 50 mg of white powdery crystal was obtained (yield = 45.5 %, melting point = 102 - 105 °C).
TLC: Rf = 0.22 (Kieselgel 60 $F_{254}$, Merck product, toluene/acetone = 1/1)

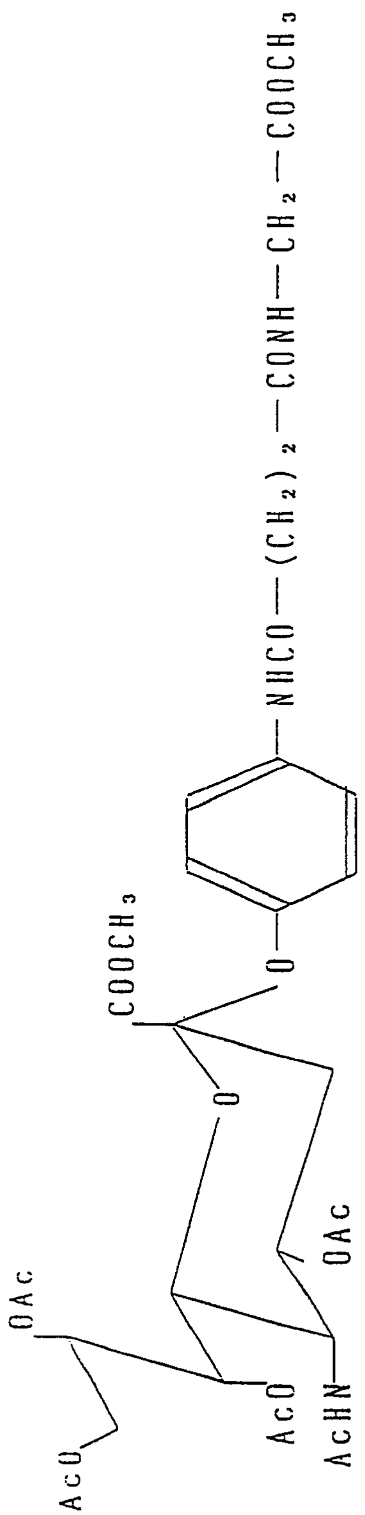

[Physical properties of the product]

$C_{33}H_{43}O_{17}N_3$
FAB-MS m/z : 754 ($M^+$ + 1)

$^1H-NMR^{ppm}_{500MHz}$(CDCl$_3$, TMS)

1.898 (3H, s, -NHCOCH$_3$),
2.034;2.054;2.118;2.133 (all 3H, all s, -OCOCH$_3$ X 4),
2.175 (1H, t, J=12.5Hz, $H_{3ax}$),
2.65 - 2.71 (5H, m, -CH$_2$-CH$_2$- +$H_{3eq}$),
3.648 (3H, s, 2 -COOCH$_3$),
3.749 (3H, s, -NHCH$_2$COOCH$_3$),
4.946 (1H, ddd, J=12.5, 10.3, 4.8Hz, H-4),
7.012 (2H, d, J=9.2Hz, phenyl-H),
7.417 (1H, d, J=9.2Hz, phenyl-H),
IR$\nu^{KBr}_{max}$ cm$^{-1}$ : 3300, 1750, 1660, 1550,

[13-2] The second method (mixed acid anhydride process)

107.4 mg of 4′-[(methyl 5-acetamido-4, 7, 8, 9-tetra-O-acetyl-3, 5-dideoxy-α-D-glycero-D-galacto-2-nonulopyranosylonate)oxy] succinanilic acid obtained in the aforementioned Embodiment 6, and 20.4 μl of triethylamine were dissolved in 2 ml of anhydrous tetrahydrofuran, and with cooling to -12 °C and stirring, 18.9 μl of isobutylchloroformate were added and allowed to react for 10 minutes. Then, 1 ml of anhydrous chloroform solution dissolving 18.3 mg of glycine methyl ester hydrochloride and 20.4 μl of triethylamine was added to the mixture of the above-mentioned mixed acid anhydride solution. After stirring for a while, the solution was allowed to react at 0 °C for 1 hour, then at room temperature with stirring for one day. After reaction solvent was removed, the residue obtained was applied with silica gel column chromatography (Wakogel C = 300, CHCl$_3$/MeoH = 20/1) to give fractional solution containing the object. Solvent was removed from the obtained fractional solution and 36.3 mg of white powdery crystal was obtained (yield = 30.6 %).
TLC: Rf = 0.22 (Kieselgel 60 $F_{254}$, Merck product, toluene/acetone = 1/1)
Rf = 0.34 (Kieselgel 60 $F_{254}$, Merck product, chloroform/methanol = 20/2)
1H-NMR, IR data agreed with that obtained with the aforementioned first method (active ester process). even when solvents used in the above-mentioned reactions (tetrahydrofuran, chloroform) were replaced with dimethylformamide, the similar object substance was able to obtain.

[13-3] The third method

37.4 mg of 4′-[(methyl 5-acetamido-3, 5-dideoxy-α-D-glycero-D-galacto-2-nonulopyranosylonate) oxy] succinaniloyl) glycine methyl ester and 0.8 ml of acetic anhydride were dissolved in 0.8 ml of anhydrous pyridine and allowed to react at room temperature for one day with stirring. Reaction solvent was removed and the residue obtained was applied with silica gel column chromatography (Wakogel C = 300, CHCl$_3$/MeOH = 20/1) to give fractional solution containing the object. Solvent was removed from the obtained fractional solution and 28.8 mg of white powdery crystal (yield = 59.8 %) was obtained.
TLC: Rf = 0.22 (Kieselgel 60 $F_{254}$, Merck product, toluene/acetone = 1/1)
Rf = 0.34 (Kieselgel 60 $F_{254}$, Merck product, chloroform/methanol = 20/1)
1H-NMR data completely agreed with that obtained with the aforementioned first method (active ester process).

[Embodiment 14]

Synthesis of 4′-[(methyl 5-acetamido-4, 7, 8, 9-tetra-O-acetyl-3, 5-dideoxy-α-D-glycero-D-galacto-2-nonulopyranosylonate)oxy] succinanilohylhydrazide:

100 mg of 4′-[(methyl 5-acetamido-4, 7, 8, 9-tetra-O-acetyl-3, 5-dideoxy-α-D-glycero-D-galacto-2-nonulopyranosylonate)oxy] succinanilic acid obtained in the aforementioned Embodiment 6, 38 mg of N, N′-disuccinimidyl carbonate, and 11.7 μl of anhydrous pyridine were dissolved in 10 ml of anhydrous acetonitrile and allowed to react at room temperature for 8 hours. After disappearance of raw material was

confirmed with a TLC, while cooling, 10 µl of anhydrous hydrazine was added and allowed to react for one day. Reaction solvent was removed and the residue obtained was applied with gel filtration chromatography (LH-20, MeOH), then further with silica gel chromatography (Wakogel C-300, $CHCl_3$/MeOH = 10/1) to give fractional solution containing the object. Solvent was removed from the obtained fractional solution and 40.5 mg of white powdery crystal (yield = 40 %, melting point = 110 - 113 °C) was obtained.
Rf = 0.52 (Kieselgel 60 $F_{254}$, Merck product, ethyl acetate/methanol = 5/3)
Rf = 0.20 (Kieselgel 60 $F_{254}$, Merck product, chloroform/methanol = 10/1)

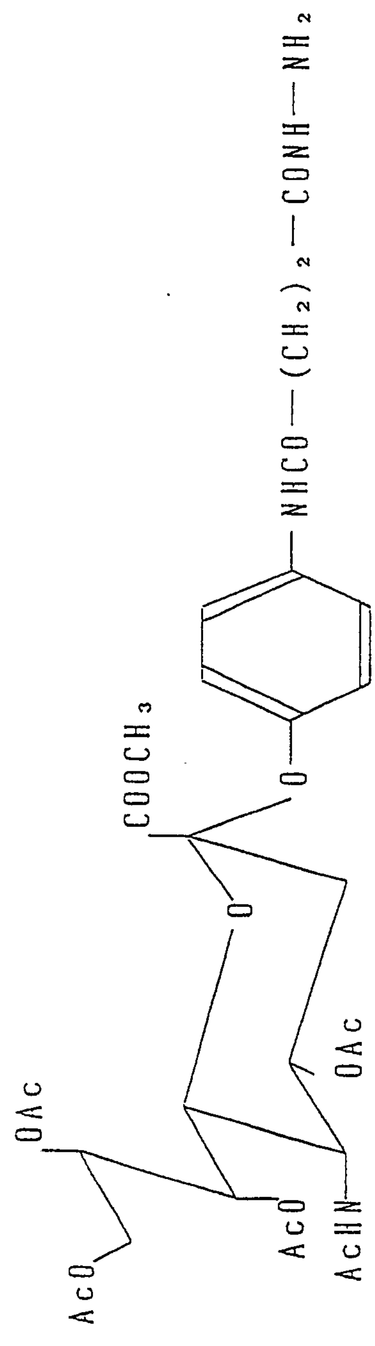

[Physical properties of the product]

Element analysis $C_{30}H_{40}O_{15}N_4$
FAB-MS m/z : 697 ($M^+$ + 1)

$^1H$-NMR $^{ppm}_{500MHz}$ ($CDCl_3$, TMS)

1.811 (3H, s, $-NHCOCH_3$),
1.972;2.031;2.055 (6H;3H;3H, all s, $-OCOCH_3$ X 4),
2.108 (1H, t, J=12.5Hz, $H_{3ax}$),
2.492 (4H, m, $-CH_2-CH_2-$),
2.622 (3H, m, $-\overline{CH_2}-CH_2-$ $+H_{3eq}$),
3.544 (3H, s, 2 $-CO\overline{OCH_3}$),
4.876 (1H, m, H-4),
6.921 (2H, d, J=8.8Hz, phenyl-H),
7.355 (2H, d, J=8.8Hz, phenyl-H),
IR$\nu^{KBr}_{max}$ $cm^{-1}$ : 3300, 1740, 1660, 1540,

[Embodiment 15]

Synthesis of N-4′-[methyl (5-acetamido-3, 5-dideoxy-α-D-glycero-D-galacto-2-nonulopyranosylonate)oxy] succinaniloyl) glycine methyl ester:

98 mg of 4′-[(methyl 5-acetamido-3, 5-dideoxy-α-D-glycero-D-galacto-2-nonulopyranosylonate)oxy] succinanilic acid obtained in the aforementioned Embodiment 9 and 27.1 μl of triethylamine were dissolved in 1 ml of anhydrous dimethylformamide and 25 ml of iso-butylchloroformate were added with stirring and cooling (-15 °C), then allowed to react for 10 minutes. Then, 1 ml of anhydrous dimethylformamide solution dissolving 24.4 mg of glycine methyl ester hydrochloride salt and 27.1 μl of triethylamine was added to the above-mentioned mixture acid anhydride solution. After stirring for a while, the solution was allowed to react at 0 °C for 1 hours, then at room temperature with stirring for one day. After reaction solvent was removed under the depressurizing condition, the residue obtained was applied with $C_{18}$-column chromatography (YMC.GEL ODS 60 Å 60/200 mesh). The solution was eluted first with water, then with water/methanol = 1/1 solution, and the fractional solution containing the object was freeze-dryed to give 50 mg of white powdery crystal (yield = 45 %, melting point = 111 - 113 °C).
TLC: Rf = 0.46 (Kieselgel 60 $F_{254}$, Merck product, $CHCl_3/MeOH/ACOH$ = 6/3/0.5)

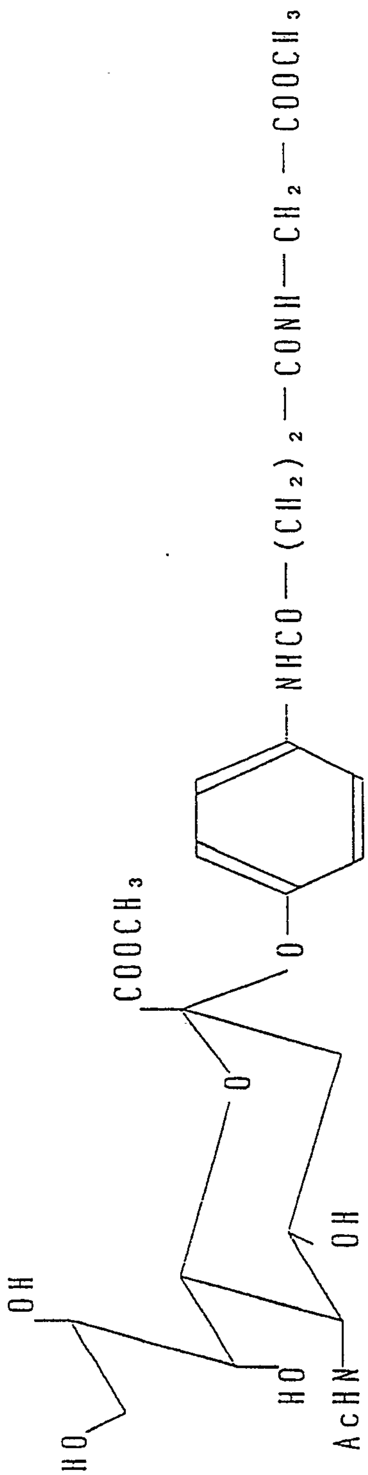

[Physical properties of the product]

$C_{25}H_{35}O_{13}N_3$
FAB-MS m/z : 586 ($M^+$ + 1)

$^{1}H\text{-}NMR^{ppm}_{500MHz}$ (D2O, TSP)

2.040 (1H, t, J = 12.5Hz, $H_{3ax}$),
2.051 (3H, s, -$NHCOCH_3$),
2.68 - 2.74 (4H, m, -$CH_2$-$CH_2$-),
2.890 (1H, dd, J = 12.8, 4.4Hz, $H_{3eq}$),
3.726 (3H, s, 2-$COOCH_3$),
3.763 (3H, s, -$NHCH_2COOCH_3$),
7.164 H, d, J = 8.8Hz, phenyl-H),
7.397 (2H, d, J = 8.8Hz, phenyl-H),
$IR\nu^{KBr}_{max}$ $cm^{-1}$ : 3350, 1740, 1650, 1550,

[Embodiment 16]

Synthesis of N-4′-[methyl (5-acetamido-4, 7, 8, 9-tetra-O-acetyl-3, 5-dideoxy-α-D-glycero-D-galacto-2-non-ulopyranosylonate)oxy] succinaniloyl) glycine:

100 mg of 4′-[(methyl 5-acetamido-4, 7, 8, 9-tetra-O-acetyl-3, 5-dideoxy-α-D-glycero-D-galacto-2-non-ulopyranosylonate) oxy] succinanilic acid obtained in the aforementioned Embodiment 6 and 20.4 μl of triethylamine were dissolved in 2 ml of anhydrous tetrahydrofuran, and 18.9 μl of isobutylchloroformate were added with stirring and cooling (-15 °C), then allowed to react for 10 minutes. Then, the above-mentioned mixture acid anhydride solution was added to 1 ml of tetrahydrofuran-water (1:1) mixture solution dissolving 11 mg of glycine and 20.4 μl of triethylamine with cooling (0 °C) and stirring. The solution was allowed to react at 0 °C for 1 hour, then at room temperature with stirring for one day. After reaction solvent was removed under the depressurizing condition, the residue obtained was freeze-dryed. The methanol solution of the residue obtained was made acidic with Dowex 50w ($H^+$), then solvent was removed.

The residue was applied with silica gel column chromatography (Wakogel C-300, acetate/methanol = 5/3) to give the fractional solution containing the object. Solvent was removed from the fractional solution and 54 mg of white powdery crystal were obtained (yield = 50 %, melting point = 105 - 108 °C).

TLC: Rf = 0.15 (Kieselgel 60 $F_{254}$, Merck product, acetate/methanol = 5/3)

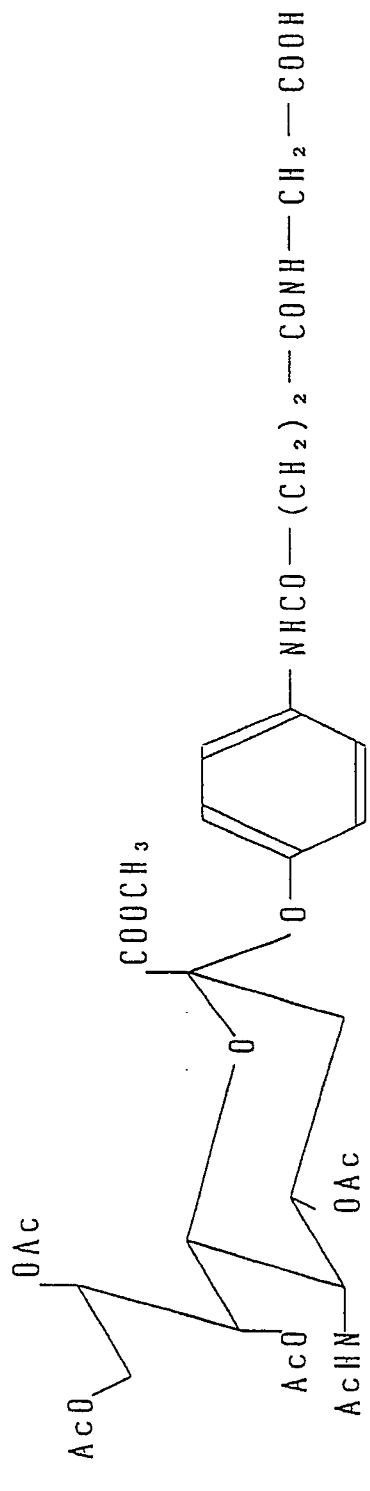

[Physical properties of the product]

$C_{32}H_{41}O_{17}N_3$
FAB-MS m/z : 740 ($M^+$ + 1)

$^1H$-NMR $^{ppm}_{500MHz}$ ($CDCl_3$, TMS)

1.880 (3H, s, $-NHCOCH_3$),
2.0241;2.085;2.117 (all s, $-OCOCH_3$ X 4),
3.605 (3H, s, $2-COOCH_3$),
3.61 (2H, broad s, $-NH-\underline{CH_2}-COOH$),
4.93 (1H, m, H-4),
6.962 (2H, d, J=8.1Hz, phenyl-H),
7.412 (2H, d, J=8.1Hz, phenyl-H),
$IR\nu_{max}^{KBr}$ $cm^{-1}$ : 3350, 1740, 1660, 1540,

## Claims

1 . Sialic acid derivative with active ester groups expressed by the formula [I]

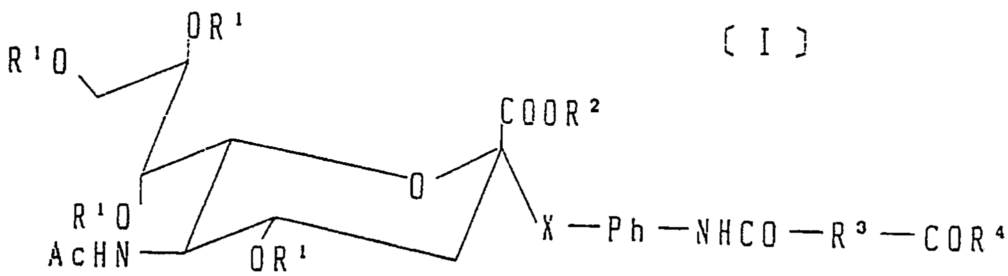
[ I ]

where $R^1$ denotes hydrogen or acetyl group, $R^2$ hydrogen or lower alkyl group, $R^3$ $C_2H_4$, $C_3H_6$ or $C_2H_2$, $R^4$ hydroxyl group, residue left after removing hydrogen from the alcohol portion of active ester or alkyloxycarbonyloxy group, Ac acetyl group, Ph phenyl group, X oxygen or sulfur.

2. Sialic acid derivative with active ester groups according to claim 1; the alcohol portion of the said active ester is either one specie selected from the groups of N-hydroxysuccinimide, N-hydroxy-5-norbornene-2, 3-dicarboximide, N-hydroxyphthalimide, N- hydroxybenzotriazole, p-nitrophenol, 2, 4-dinitrophenol, 2, 4, 5-trichlorophenol, or pentachlorophenol.

3. Sialic acid derivative with active ester groups according to claim 1; the alkyl group of alkyloxycarbonyloxy group is either one specie selected from the groups of methyl group, ethyl group, n-butyl group, isobutyl group, phenyl group, and benzyl group.

4. Biological half-life extender of biologically active substance comprising sialic acid derivatives containing active ester groups according to claim 1 , 2, or 3.

5. Sialic acid derivative expressed by the formula [II],

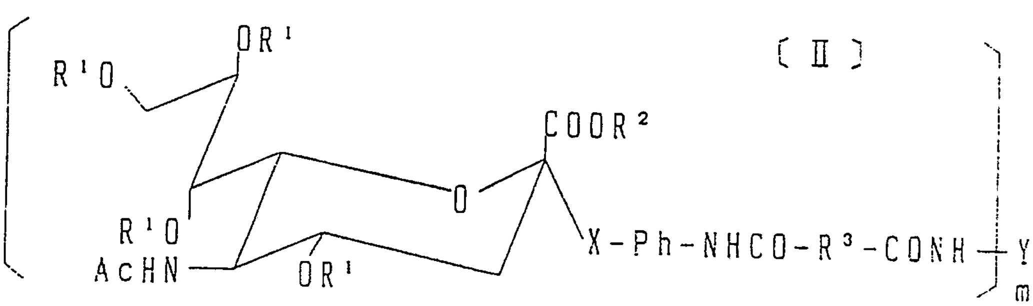
[ II ]

where $R^1$ denotes hydrogen or acetyl group, $R^2$ hydrogen or lower alkyl group, $R^3$ $C_2H_4$, $C_3H_6$ or $C_2H_2$, Ac acetyl group, m 1-60, Ph phenyl group, X oxygen or sulfur, Y the residue left from removing a number of amino group from amino compounds.

6. Sialic acid derivative according to claim 5 in which the amino compounds include amino acids and their derivatives.

7. Sialic acid derivative expressed by the formula [III],

[ III ]

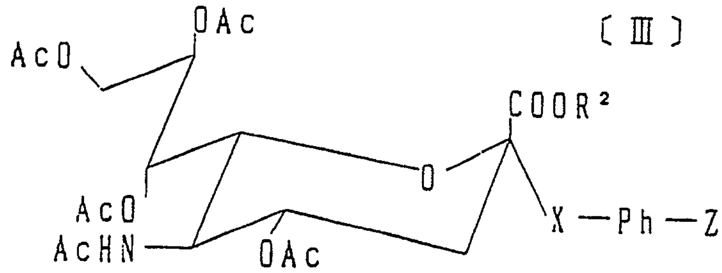

where $R^2$ hydrogen or lower alkyl group, Ac acetyl group, m 1-60, Ph phenyl group, X oxygen or sulfur, Z a specie selected from the groups comprising nitro group, amino group, N, N'-dialkyl-substituted amino group and N-succinimide group, unless Z is a nitro group when X is oxygen.

Claims for the following Contracting State : ES

1 . A preparation process of sialic acid derivative expressed by the formula [I]

[ I ]

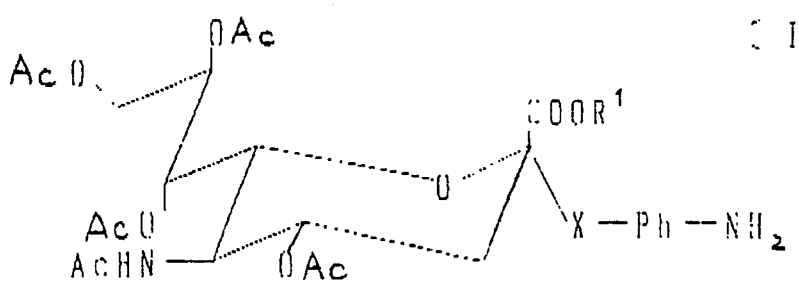

where Ac denotes acetyl group, $R^1$ denotes lower alkyl group, and Ph denotes phenyl group, comprising: reacting a sialic acid derivative expressed by the formula [II]

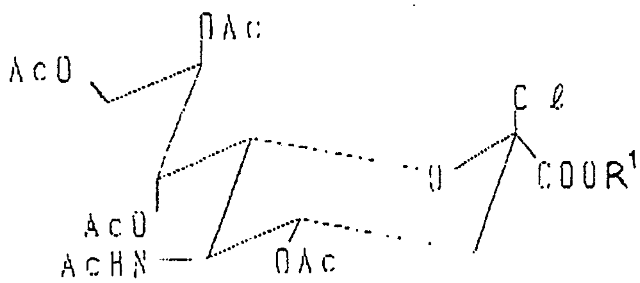

[II]

where Ac and $R^1$ are same as above, with anhydrous salt of p-nitrophenol or anhydrous salt of p-nitrothiophenol in a polar solvent, to obtaine a sialic acid derivative expressed by the formula [III]

[III]

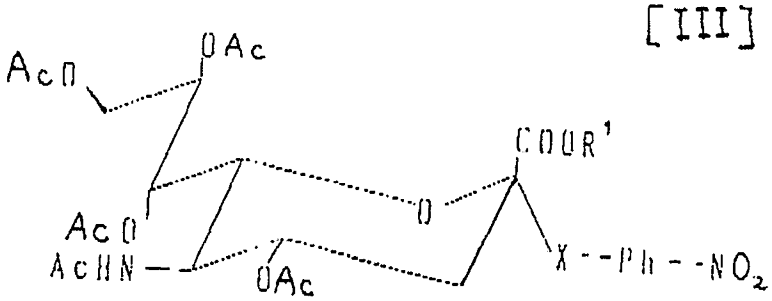

where X denotes oxygen or sulfur, Ph and $R^1$ are same as before,

and then,
adding hydrogen, in the presence of Pd/C, to said sialic acid derivative expressed by the formula [III].

2. A preparation process of a sialic acid derivative with active ester groups, expressed by the formula [IV]

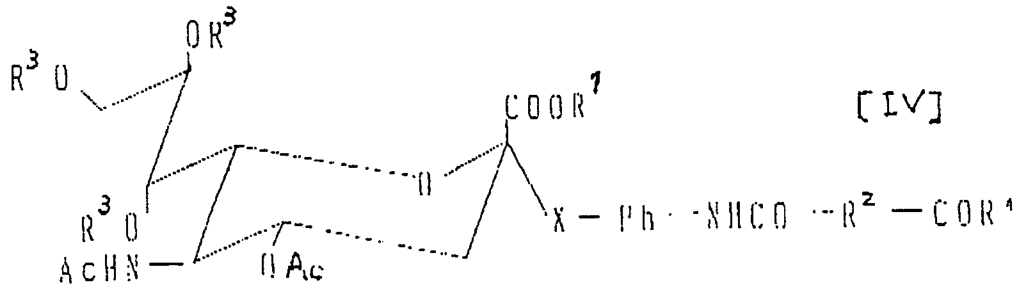

[IV]

where $R^2$ denotes $C_2H_2$, $C_2H_4$ or $C_3H_6$, $R^3$ denotes hydrogen or acetyl group, $R^4$ denotes hydroxyl group, residue left after removing hydrogen from the alcohol portion of active ester, or alkyloxycarbonyloxy group, and $R^1$, Ac, Ph and X are same as defined in claim 1 comprising:
reacting said sialic acid derivative [I] in claim 1 with acid anhydride in a polar solvent, to obtaine a sialic acid derivative expressed by the formular [V]

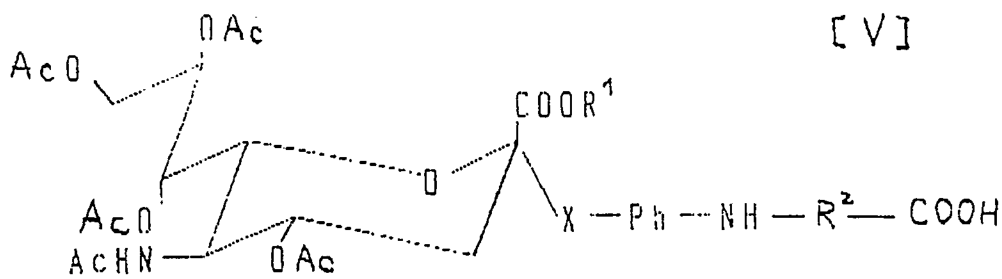

[V]

where and Ac, Ph, $R^1$ and X are same as above,
and then,
reacting said sialic acid derivative [V] with a compound which contains active estér group or a compound which is condensated with COOH of said sialic acid derivative [V] to produce active ester group, in a polar solvent.

3. A preparing process of sialic acid derivative according to claim 2; wherein,
the alcohol portion of the said active ester is either one specie selected from the groups of N-hydroxysuccinimide, N-hydroxy-5-norbornene-2, 3-dicarboximide, N-hydroxyphthalimide N-hydroxybenzotriazole, p-nitrophenol, 2, 4-dinitrophenol, 2, 4, 5-trichlorophenol or pentachlorophenol.

4. Biological half-life extender of biologically active substance comprising sialic acid derivative obtained by the process of claim 2 or 3.

5. A preparing process of sialic acid derivative expressed by the formula [VI]

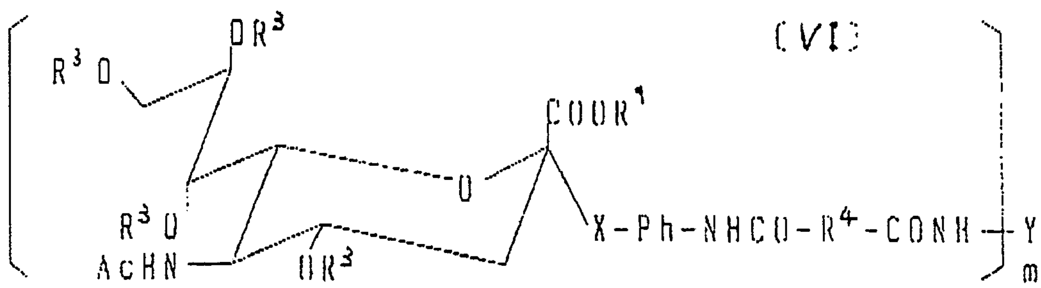

[VI]

wherein Ac denotes acetyl group, m is 1 - 60, Ph is phenyl group, $R^1$ is hydrogen or lower alkyl group, $R^3$ is hydrogen or acetyl group, $R^4$ is $C_2H_2$, $C_2H_4$ or $C_3H_6$, X is oxygen or sulfur, Y denotes residue left from removing m number of amino group from amino compounds, comprising:
reacting said sialic acid derivative with active ester groups, expressed by the formula [IV] in claim 2 with amino compounds.

6. Preparing process according to claim 5,
wherein the amino compounds include amino acids or their derivatives.